(19) **Europäisches Patentamt**
**European Patent Office**
**Office européen des brevets**

(11) **EP 1 930 725 A1**

(12) **EUROPEAN PATENT APPLICATION**
published in accordance with Art. 153(4) EPC

(43) Date of publication:
**11.06.2008 Bulletin 2008/24**

(21) Application number: **06810916.4**

(22) Date of filing: **29.09.2006**

(51) Int Cl.:
***G01N 33/543*** *(2006.01)*     ***G01N 33/49*** *(2006.01)*

(86) International application number:
**PCT/JP2006/319537**

(87) International publication number:
**WO 2007/037409 (05.04.2007 Gazette 2007/14)**

(84) Designated Contracting States:
**CH DE ES FR GB IT LI**

(30) Priority: **30.09.2005 JP 2005285900**

(71) Applicant: **Pulse-Immunotech Corporation**
**Hachioji-shi, Tokyo 192-0982 (JP)**

(72) Inventor: **IWATA, Keisuke**
**Saitama 346-0011 (JP)**

(74) Representative: **Grünecker, Kinkeldey,**
**Stockmair & Schwanhäusser**
**Anwaltssozietät**
**Leopoldstrasse 4**
**80802 München (DE)**

(54) **METHOD OF ASSAYING SUBSTANCE WITH AFFINITY IN SAMPLE CONTAINING BLOOD-CELL INGREDIENT**

(57)    In the methods for measuring affinity substances using pearl chain formation of carrier particles, the use of carrier particles having a particular particle diameter enables carrier particles to be specifically counted even if blood cell components coexist. Whole blood samples can be used as samples to be measured without separating serum and plasma. Therefore, there is no need to separate serum or plasma when analyzing blood cell components.

**Description**

Technical Field

The present invention relates to methods for measuring substances having affinity (also referred to as "affinity substances") using agglutination reactions of carrier particles.

Background Art

Conventional methods for detecting or measuring the presence of biologically specific reactive substances include, for example, enzyme immunoassays and radioimmunoassays. These are highly sensitive and accurate methods. However, reagents used in these methods are unstable because enzymes or radioisotopes are used as labels. Furthermore, these assays that use radioisotopes require meticulous attention to detail and technical skills because there are regulations for radioisotope storage and preservation. Thus, there has been a need for more convenient measurement methods. Furthermore, since these methods require a relatively long time for measurement, they cannot be applied for urgent tests. Under these circumstances, extensive studies on rapid and highly sensitive measurement methods were begun.

Since 1970, analysis methods that use agglutination of carrier particles as an indicator for measuring immunological reactions have been put into practical use. In these methods, quantitative analysis is enabled by optical measurement of the degree of carrier particle agglutination. The optical methods that use latex particles as a carrier particle for measuring immunological particle agglutination reactions are called latex agglutination turbidimetry. In general, the reaction temperature in these analysis methods ranges from 37 to 45°C, and specific agglutination reactions proceed upon mixing with a stirring impeller or such. Since the time required for measurement (reaction) ranges from about 10 to 20 minutes, these methods are more rapid than enzyme immunoassays or radioimmunoassays. However, these methods are said to be inferior to enzyme immunoassays or such in sensitivity and measurement range.

Methods for determining the particle size distribution in latex agglutination methods are also known (Non-patent Document 1, Cambiaso CL, J. Immunol. Methods 18(1-2), 33-44, 1977; Non-patent Document 2, Matsuzawa et al., Kagaku to kogyo (Chemistry and Chemical Industry), Vol. 36, No. 4, pp. 206-208, 1983). In latex agglutination turbidimetry, light transmittance through particle suspensions is determined by measuring the state and the number of dispersed individual particles by methods that determine particle size distribution. In the report of Cambiaso et al., an antigen was reacted with a reagent of antibody-bound latex particles (0.8 $\mu$m diameter) at 37°C for 20 minutes. The particles were counted after the reaction and the antigen was quantified based on the level of decrease in the number of particles due to agglutination. The number of particles was determined using a counter that is based on the principle of laser light scattering.

Meanwhile, Matsuzawa et al. incubated an antigen with a reagent of antibody-bound latex particles (1 $\mu$m diameter) for 6 hours. After the reaction, mean particle volume was determined by an electric resistance method to quantify the antigen. However, only the PAMIA system (SYSMEX CORPORATION), which uses a laser scattering sheath flow method, has been put into practical use and is widely used. PAMIA uses latex particles that have a diameter of 0.78 $\mu$m. Immunoassay is carried out by counting latex particles after a 15-minute reaction at 45°C. PAMIA is more sensitive than latex agglutination turbidimetry. However, PAMIA is said to be inferior in sensitivity when compared to high sensitivity immunoassay methods such as radioimmunoassays (RIA) and enzyme immunoassays (EIA).

In general, latex agglutination turbidimetry uses latex particles that have a diameter of 0.05 to 0.6 $\mu$m. When such small particles are used, methods for analyzing particle size distribution in latex agglutination are easily affected by substances that interfere with measurement. For example, lipids, proteins, blood cell components, and such coexist in body fluids such as blood and urine. These coexisting substances are indistinguishable from carrier particles, and may lead to inaccurate counting of carrier particles. Hence, relatively large particles have been used to avoid the impact of interfering substances of measurement. In contrast, agglutination reactions hardly take place when particles having a diameter of about 1 $\mu$m, such as those in Matsuzawa et al. are used. This is the reason why latex particles with a diameter of about 0.8 $\mu$m have so far been used. The diameter of the aperture (small hole) that Matsuzawa et al. used to measure mean particle volumes was 30 $\mu$m. Apertures of this size are more susceptible to clogging. However, 0.8 tol $\mu$m particles cannot be detected when the aperture diameter is greater than 30 $\mu$m.

Furthermore, a method of applying an alternating voltage to a reaction system to accelerate biologically specific agglutination reactions and to facilitate detection of the agglutinates formed is known (Patent Document 1 / Japanese Patent Application Kokai Publication No. (JP-A) H07-83928 (unexamined, published Japanese patent application)). This method uses biologically specific agglutination reactions of carrier particles for detecting or measuring the presence of biologically specific reactive substances, and comprises applying an alternating voltage to the reaction system to produce an electric field intensity of 5 to 50 V/mm in the presence of a salt at 10 mM or higher.

Carrier particles in an electric field are aligned along the electric field (pearl chain formation). The aligned carrier particles redisperse when the electric field is terminated. When a biologically specific reactive substance is present during pearl chain formation, the carrier particles do not redisperse and pearl chain-like carriers are found even after the electric field is terminated. The above-described measurement uses this phenomenon. Specifically, reactions of biologically specific reactive substances are promoted in an electric field. The reaction products can be detected by allowing carrier particles to redisperse after the termination of the electric field. The accuracy of the immunological measurement method using pearl chain formation was further improved by counting carrier particles based on their three-dimensional information as an indicator (Patent Document 2 / PCT pamphlet WO2004/111649).

[Patent Document 1] JP-A H07-83928
[Patent Document 2] WO2004/111649
[Patent Document 3] JP-AH10-48214
[Patent Document 4] JP-A 2002-107365
[Patent Document 5] WO2001/96868
[Non-Patent Document 1] Cambiaso CL, J. Immunol. Methods. 1977; 18(1-2):33-44.
[Non-Patent Document 2] Matsuzawa, et al. Chemistry and Chemical Industry, Vol. 36, No. 4, p206-208, 1983.

Disclosure of the Invention

[Problems to be Solved by the Invention]

Blood sample analyses, as well as methods using carrier particles, generally use serum or plasma samples in which blood cell components have been separated. One of the reasons for this is that blood cell components interfere with measurements. For example, when the agglutination of carrier particles is optically detected, the presence of colored components such as erythrocytes in the samples may interfere with the measurement results. Blood cell components may also be mistakenly counted as carrier particles in methods including a step of counting particles, such as immunoassays utilizing pearl chain formation.

The separation of blood cell components requires centrifugation or use of filters. However, if an immunoassay method that allows the use of whole blood as a sample is provided, such operations can be omitted. In particular, if a method that allows the use of whole blood as a sample is achieved, it would be useful in urgent examinations.

To solve these problems, methods of optically measuring the agglutination of carrier particles using hemolyzed whole blood samples were suggested (JP-A H10-48214). These methods use surfactants as hemolytic agents. However, surfactants are likely to inhibit immunological reactions. On the other hand, in the method of hemolyzing samples by adding a hemolytic agent after the immunological reaction (JP-A 2002-107365), the effect of surfactants on the immunological reaction might be small. However, it is undeniable that cell components produced by hemolysis can interfere with optical measurements.

Furthermore, it is known that the use of whole blood samples have been attempted in the method for optically counting agglutinated and unagglutinated particles by employing carrier particles smaller than blood cells (PCT pamphlet WO2001/96868; Japanese Patent Kohyo Publication No. (JP-A) 2004-503779 (unexamined Japanese national phase publication corresponding to a non-Japanese international publication)). In this method, no hemolytic agents are used. Methods that allow the use of whole blood samples in immunoassays using pearl chain formation would also be useful.

Thus, an objective of the present invention is to provide methods for using samples containing blood cell components in methods for measuring affinity substances using pearl chain formation.

[Means for Solving the Problems]

In order to solve the above problems, the present inventors carried out a large number of studies on measurement methods that are less affected by interference from blood cell components. As a result, they discovered that agglutinated or unagglutinated carrier particles can be specifically counted even in the presence of blood cell components by selecting carrier particles having a certain particle diameter, thereby completing the present invention. More specifically, the present invention relates to the following measurement methods:

[1] a method for measuring an affinity substance, which comprises the steps of (1) to (3) or (1') to (3'):

(1) applying a voltage pulse to a reaction solution in which a target affinity substance is mixed with carrier particles which are bound to a binding partner having an activity to bind to the target affinity substance;
(2) counting either or both of agglutinates of carrier particles formed due to binding of the target affinity substance, and/or unagglutinated carrier particles which are not bound to the target affinity substance, based on their three-dimensional information as an indicator; and

(3) determining the level of the target substance based on either or both of the level of agglutinate formation and/or the level of unagglutinated carrier particles; or

(1') applying a voltage pulse to a reaction solution in which an agglutination reagent component is mixed with a target affinity substance and carrier particles which are bound to a binding partner having an activity to bind to the target affinity substance, wherein the carrier particles agglutinate due to the agglutination reagent, and the agglutination is inhibited by the target affinity substance;

(2') counting either or both of agglutinates of carrier particles formed due to binding of the agglutination reagent, and/or carrier particles whose agglutination is inhibited by binding of the target affinity substance, based on their three-dimensional information as an indicator; and

(3') determining the level of the target substance based on either or both of the level of agglutinate formation and/or the level of unagglutinated carrier particles,

wherein the target affinity substance is included in a medium comprising blood cell components,

wherein step (1) or (1') is carried out in the presence of said medium, and wherein the carrier particles in step (1) or (1') are of a size that yields agglutinates having a volume that can be distinguished from blood cell components;

[2] the method of [1], wherein the volume that can be distinguished from blood cell components differs from a volume of 40 $\mu m^3$ to 100 $\mu m^3$ by at least 10% or more;

[3] the method of [2], wherein the average diameter of the carrier particles is 0.5 $\mu m$ to 2.4 $\mu m$ or 6 $\mu m$ to 20 $\mu m$;

[4] the method of [3], wherein the average diameter of the carrier particles is 1 $\mu m$ to 2 $\mu m$;

[5] the method of [1], wherein the voltage pulse is applied under a condition that does not substantially disrupt the blood cell components included in the sample;

[6] the method of [5], wherein the voltage pulse is an alternating voltage which gives an electrolytic intensity of 10 V/mm to 50 V/mm;

[7] the method of [6], wherein the voltage pulse is an alternating voltage which gives an electrolytic intensity of 15 V/mm to 30 V/mm;

[8] the method of [1], wherein the voltage pulse is an alternating voltage and its frequency is 100 KHz to 10 MHz;

[9] the method of [8], wherein the voltage pulse is an alternating voltage and its frequency is 400 KHz to 1 MHz;

[10] the method of [1], wherein the three-dimensional information of the agglutinates or carrier particles is physically measured in step (2) or (2');

[11] the method of [10], wherein a method of physically measuring the three-dimensional information is selected from the group consisting of electric resistance method, laser diffraction/scattering method, and three-dimensional image analysis method;

[12] the method of [1], wherein the carrier particles are counted after the electric field is terminated in step (2) or (2');

[13] the method of [12], which further comprises the step of diluting the carrier particles after the electric field is terminated in step (2) or (2');

[14] the method of [1], wherein the voltage pulse is applied multiple times;

[15] the method of [14], which comprises the steps of applying a voltage pulse, dispersing the carrier particles, and then applying a subsequent voltage pulse;

[16] the method of [14], wherein the multiple voltage pulses are in different directions;

[17] the method of [1], wherein the concentration of the carrier particles in the reaction solution is 0.1 to 1 w/v%; and

[18] the method of [17], wherein the concentration of the carrier particles in the reaction solution is 0.2 to 0.5 w/v%.

[Effects of the Invention]

The present invention provides methods that can measure affinity substances in samples containing blood cell components by immunoassays using pearl chain formation. In the present invention, affinity substances can be measured even in the presence of blood cell components. Therefore, whole blood samples can be used to measure affinity substances in the blood, without separating blood cell components. Separation of blood cell components is a time-consuming and laborious operation. Therefore, for example, it will be useful in urgent examinations to provide measurement methods that allow the use of whole blood samples.

Meanwhile, in immunoassays that utilize pearl chain formation, immunological agglutination of carrier particles is enhanced by an electric field. As a result, efficient immunological reactions can be achieved using relatively larger carrier particles, which are considered inappropriate for agglutination reactions under normal conditions. The use of large carrier particles contributes to improving the accuracy of measurements. In addition, immunoassays that utilize pearl chain formation can detect affinity substances using a small amount of samples, in a short period of time, and with high sensitivity. Therefore, the present invention, which has achieved measurement methods that use whole blood samples through immunoassays using pearl chain formation, has great significance.

Furthermore, in the measurement methods of the present invention, carrier particles can be specifically counted even in the presence of blood cell components. Therefore, in the present invention, there is no need to disrupt blood cell components. Accordingly, the present invention does not need hemolytic agents, which may interfere with immunological reactions.

Furthermore, in a preferred embodiment of the present invention, the serum concentration of measured substances can be determined by correcting measured values with the volume of blood cell components. The proportion of blood cell components in a whole blood sample varies greatly among subjects. Even the same subject shows a wide range of fluctuation in the proportion of blood cell components. For example, even if the serum concentration is not different, variations in the blood cell component proportions cause differences in the concentration of substances in the whole blood. That is, the whole blood concentration of substances in a subject with a larger amount of blood cell components is lower than that in a subject with smaller amount of blood cell components. Therefore, substance concentrations in the blood are often compared based on the serum concentrations. Since serum concentration is a value which is free from the effect of the proportion of blood cell components, such values can be compared more accurately among subjects.

In a preferred embodiment of the present invention, it is possible to determine the volume of blood cell components included in the reaction solution, as well as to count the number of carrier particles. The serum concentration of a measured substance can be determined by correcting the measured value for the substance based on the determined volume of blood cell components. That is, in a preferred embodiment of the present invention, target substances to be measured can be analyzed without serum separation processes, and their serum concentration can also be determined, even when whole blood is used as a sample.

Brief Description of the Drawings

Fig. 1 depicts the result of determining the volume of blood cell components included in whole blood. In the figure the vertical axis shows the number of particles counted and the horizontal axis shows the volume of particles.

Fig. 2 (A) depicts the configuration of an apparatus based on the present invention; and (B) depicts a cross section of the pulsing chamber.

Fig. 3 depicts the result of performing an immunoassay on a whole blood sample in which the antigen-antibody reaction was promoted by pearl chain formation according to the method of the present invention. In the figure, the vertical axis shows the number of particles counted, and the horizontal axis shows the volume of the particles.

Fig. 4 depicts the result of performing an immunoassay on a whole blood sample without carrying out pearl chain formation. In the figure, the vertical axis shows the number of particles counted, and the horizontal axis shows the volume of the particles.

Fig. 5 depicts the result of observing the disruption of blood cells when incubating at 80°C for 0 to 60 seconds. In the figure, the vertical axis shows the number of particles counted, and the horizontal axis shows the volume of the particles.

Fig. 6 depicts the result of observing the disruption of blood cells when incubating at 60°C for 0 to 90 seconds. In the figure, the vertical axis shows the number of particles counted, and the horizontal axis shows the volume of the particles.

[Descriptions of Letters or Numerals]

1: dispensing/stirring means
2: temperature control system
3: reaction chamber
4: electrodes (pulsing means)
5: diluting means
6: means for measuring particle size distribution

Best Mode for Carrying Out the Invention

The present invention relates to methods for measuring an affinity substance, which comprise the following steps (1) to (3) or (1') to (3'):

(1) applying a voltage pulse to a reaction solution in which a target affinity substance is mixed with carrier particles that are bound to a binding partner having an activity to bind to the target affinity substance;

(2) counting either or both of agglutinates of carrier particles formed due to binding of the target affinity substance, and/or unagglutinated carrier particles which are not bound to the target affinity substance, by using their three-dimensional information as an indicator; and

(3) determining the level of the target substance based on either or both of the level of agglutinate formation and/or the level of unagglutinated carrier particles; or

(1') applying a voltage pulse to a reaction solution in which an agglutination reagent component is mixed with a target affinity substance and carrier particles that are bound to a binding partner having an activity to bind to the target affinity substance, wherein the carrier particles agglutinate due to the agglutination reagent, and the agglutination is inhibited by the target affinity substance;

(2') counting either or both of agglutinates of carrier particles formed due to binding of the agglutination reagent, and/or carrier particles whose agglutination is inhibited due to binding of the target affinity substance, by using their three-dimensional information as an indicator; and

(3') determining the level of the target substance based on either or both of the level of agglutinate formation and/or the level of unagglutinated carrier particles;

wherein the target affinity substance is included in a medium containing blood cell components, wherein step (1) or (1') is carried out in the presence of said medium, and wherein the carrier particles in step (1) or (1') have a size to yield agglutinates having a volume that can be distinguished from blood cell components.

Herein, "affinity substance and binding partner having an activity to bind to the affinity substance" include all combinations of substances that can participate in a binding reaction. Specifically, when one substance binds to another substance, one is the affinity substance and the other is the binding partner. The affinity substances and binding partners of the present invention may be natural substances or artificially synthesized compounds. The affinity substances and binding partners may be purified substances or substances containing impurities. Further, the affinity substances and binding partners may exist on cellular or viral surface.

Binding reactions between the affinity substances and binding partners of the present invention include, for example, the reactions listed below. Substances that participate in these reactions can either be an affinity substance or a binding partner of the present invention.

Reaction between an antibody and an antigen or a hapten (immunological reaction);

hybridization between nucleic acids having complementary nucleotide sequences;

reaction between a lectin and its receptor;

reaction between a lectin and a sugar chain;

reaction between a ligand and its receptor;

reaction between DNA and a transcription regulatory factor.

Among the above-listed binding reactions, a preferred binding reaction of the present invention can be, for example, an immunological reaction. Antigens participating in immunological reactions include the substances listed below. These antigens include not only antigen molecules themselves but also fragments thereof, and those that are present on cell surface. These substances are only examples of antigenic substances and needless to say, the present invention is also applicable to other antigenic substances. For example, any antigenic substance that can be measured based on an immunological agglutination reaction using latex or blood cell as a carrier can be used as an affinity substance of the present invention.

Tumor markers:

AFP, CEA, CA19-9, PSA, etc. Markers of the coagulation-fibrinolytic system:

protein C, protein S, antithrombin (AT) III, FDP, FDP-D-dimer, etc.

Infection markers:

CRP, ASO, HBs antigen, etc.

Hormones:

thyroid-stimulating hormone (TSH), prolactin, insulin, etc.

Tissue components:

myoglobin, myosin, brain natriuretic peptide (BNP). hemoglobin, etc.

Others:

nucleic acids such as DNA.

Either an antigenic substance or an antibody recognizing the substance may be used as the affinity substance and the other as the binding partner. Herein, "affinity substance" refers to a target substance to be measured. On the other hand, "binding partner" refers to a substance that can be used as a probe to measure the affinity substance and has an activity to bind to the affinity substance. Thus, an antibody can be used as the binding partner when an antigen is measured. Conversely, an antibody recognizing an antigen can be used as the binding partner in the measurement of the antibody. For example, any antibody that can be measured based on an immunological agglutination reaction using latex or blood cells as a carrier can be used as an affinity substance of the present invention. Antibodies against HBs (surface antigen of hepatitis B virus), HBc (core antigen of hepatitis B virus), HCV (hepatitis C), HIV (AIDS virus), TP (syphilis), and such have been measured using immunological agglutination reactions.

Several reaction principles are known to use agglutination of carrier particles as an indicator for measuring the reaction between an affinity substance and a binding partner. Any of these reaction principles can be applied to the present invention. Examples of a measurement principle that uses agglutination of carrier particles as an indicator and applies the reaction between an affinity substance and a binding partner are described below.

Direct agglutination reaction:

The agglutination of carrier particles which results from the reaction between a target substance of measurement and its binding partner present on the carrier particles is detected. This principle is applicable, for example, to cases where an antigen molecule is measured using an antibody as the binding partner. Alternatively, the principle is also applicable when an antibody is measured as the affinity substance by using agglutination of antigen-bound carrier particles as an indicator. In general, the level of agglutination particle is directly proportional to the amount of affinity substance to be measured in a direct agglutination reaction. Specifically, the higher the level of agglutinate formation, the higher the level (namely concentration) of an affinity substance is. Conversely, when the level of unagglutinated carrier particles is high, the level (namely concentration) of an affinity substance is low.

Agglutination inhibition reaction:

The low-molecular-weight antigens called "haptens" hardly form the antigen-mediated cross-linking structure required for the agglutination of carrier particles. Therefore, haptens cannot be detected based on the principle of direct agglutination reaction. In this case, it is possible to use the agglutination reaction that results from the binding of an antibody on carrier particles to a polyhapten that comprises two or more hapten molecules or fragments comprising the epitope. A polyhapten can crosslink two or more antibody molecules and agglutinate carrier particles. However, in the presence of a hapten, the reaction between a polyhapten and an antibody is inhibited and as a result, the agglutination of carrier particles is inhibited. The level of agglutination inhibition is directly proportional to the presence of hapten. Specifically, the amount of a target substance of measurement is inversely proportional to the level of agglutination reaction. Specifically, the level (i.e., concentration) of an affinity substance is low when the level of agglutinate formation is high. Conversely, the higher the level of unagglutinated carrier particles, the higher the level (i.e., concentration) of an affinity substance is.

Target antigens of measurement that are classified as haptens include the following components.

Hormones:

estrogen, estradiol

Drugs:

Theophylline.

In the present invention, measuring a hapten based on the principle of agglutination inhibition reaction requires a component that allows the agglutination of carrier particles bound to an anti-hapten antibody. Herein, a component that allows the agglutination of carrier particles bound to an anti-hapten antibody is referred to as an "agglutination reagent". An agglutination reagent is defined as a reagent that has specific affinity for an antibody as well as activity of crosslinking carrier particles via antibody binding. The polyhapten described above can be used as an agglutination reagent in hapten measurements.

In both the direct agglutination reaction and the agglutination inhibition reaction, a standard curve or regression equation may be prepared by measuring standard samples containing a predetermined concentration of affinity substance using the same reaction system, and measuring the level of agglutinates or unagglutinated carrier particles. The level of affinity substance in a sample can be determined either from the level of agglutinate formation or the level of unagglutinated carrier particles determined in a sample measurement, using the standard curve or regression equation.

The binding partners of the present invention are used to bind carrier particles. The carrier particles of the present invention include latex particles, kaolin, colloidal gold, erythrocytes, gelatin, liposomes, and such. For latex particles, those generally used in agglutination reactions may be used. Polystyrene, polyvinyl toluene, and polymethacrylate latex particles are known. A preferred carrier particle is a polystyrene latex particle. It is possible to use latex particles that have surfaces onto which a functional group has been introduced through copolymerization of monomers having the functional group. Latex particles having a functional group, such as -COOH, -OH, -NH$_2$, or -SO$_3$, are known. A binding partner can be chemically linked to latex particles having a functional group.

In the present invention, blood cell components refer to solid components in the blood. Specifically, erythrocytes, leukocytes, platelets, or such are included in blood cell components. The number and size of each blood cell component in 1 mm$^3$ of adult blood are as follows. The number of erythrocytes in women and infants is 4.5 million and 6.9 million, respectively, which differ from that of adult men.

Erythrocytes approximately 5 million (men); size: approximately 8 $\mu$m; thickness: approximately 2 $\mu$m
Leukocytes approximately 7,500 on average; size: approximately 6 to 14 $\mu$m
Platelets approximately 140,000 to 360,000; size: approximately 2 to 3 $\mu$m (discoidal with a diameter of approximately 2 $\mu$m)

Therefore, media containing blood cell components refer to media containing such solid components present in the blood. More specifically, media containing blood cell components include the following media, for example. Among them, whole blood is a preferred medium in the present invention:

    whole blood;
    diluted whole blood; or
    fraction containing blood cell components of whole blood.

In the present invention, carrier particles having a specific particle diameter are used so that the carrier particles can be specifically counted in the presence of blood cell components. More specifically, carrier particles in the present invention are selected so that they have a size to yield agglutinates having a volume that can be distinguished from blood cell components. The distinction of volume in the present invention means that when both or either agglutinated carrier particles and/or unagglutinated carrier particles are counted based on their particle size using their three-dimensional information as an indicator, these carrier particles can be distinguished from blood cell components.

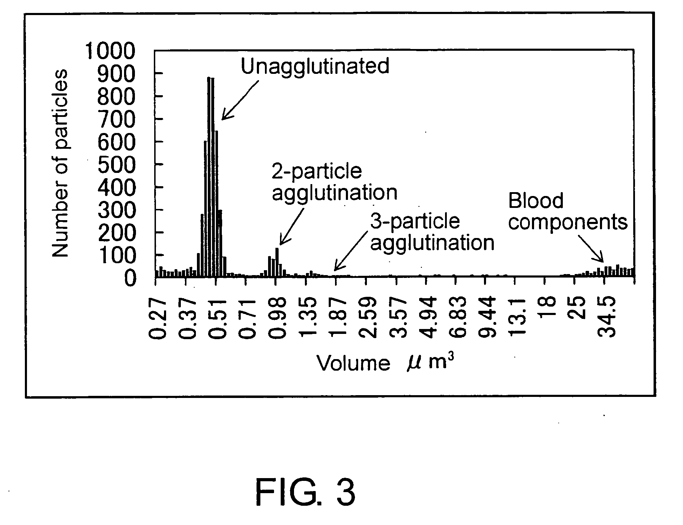

In the present invention, agglutinates are formed by agglutination of several carrier particles. More specifically, agglutinates formed by agglutination of 2 to 15 carrier particles, preferably 2 to 8 carrier particles, have sizes that can be distinguished from blood cell components. Agglutinates formed by immunological reactions in immunoassays that use pearl chain formation are mostly composed of two or three carrier particles (see Fig. 3). When a target substance has a large molecular weight or when its concentration is high, a larger number of particles may form agglutinates. For example, agglutinates composed of about five to ten carrier particles can be formed. Therefore, if agglutinates formed by agglutination of two to eight carrier particles can be distinguished from blood cell components, this means that most agglutinates can be distinguished from blood cell components. Thus, it can be said that agglutinates and blood cell components are substantially distinguishable when agglutinates formed by agglutination of two to eight carrier particles can be distinguished from blood cell components.

In the present invention, the volume that can be distinguished from blood cell components refers to, for example, a volume smaller than 40 $\mu$m$^3$. Alternatively, a volume greater than 100 $\mu$m$^3$ can also be distinguished from blood cell components. Thus, a preferred volume of agglutinates in the present invention is 40 $\mu$m$^3$ or smaller, or 100 $\mu$m$^3$ or larger. In the present invention, the distinguishable volume refers to, for example, a volume that differs from a volume of 40 $\mu$m$^3$ to 100 $\mu$m$^3$ by 10% or more, preferably 25% or more, and more preferably 50% or more.

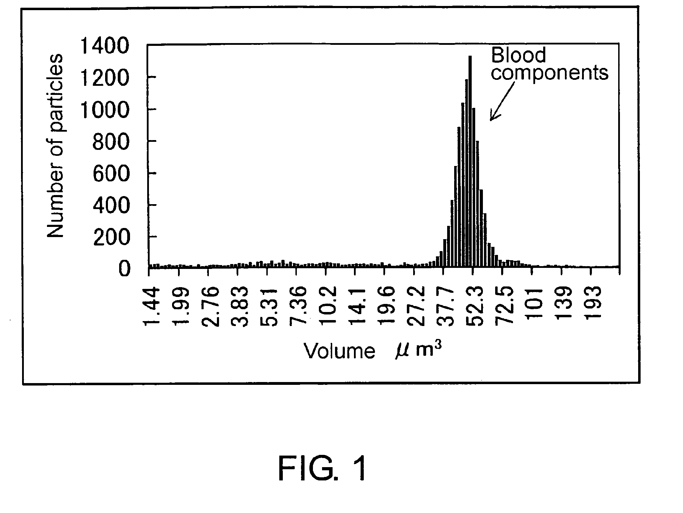

A major blood cell component having a volume of 40 $\mu$m$^3$ to 100 $\mu$m$^3$ is erythrocytes. The number of erythrocytes contained in 1 mm$^3$ of blood is approximately 5 million for adult men, approximately 4.5 million for adult women, and approximately 6.9 million for infants. Leukocytes have a slightly larger volume than erythrocytes. The number of leukocytes in 1 mm$^3$ of adult blood is as small as 7,500 on average; therefore, the majority of blood cell components is erythrocytes. An example of the results of measuring the volume distribution of whole blood using a Coulter counter calibrated with polystyrene latex beads is shown in Fig. 1. The peak found at the position of approximately 50 $\mu m^3$ corresponds to erythrocytes. A faint distribution present at a slightly larger position mainly represents a peak corresponding to leukocytes.

In order for agglutinates formed by several carrier particles to give a volume distinguishable from blood cell components, the average particle diameter of the carrier particles can be specifically chosen within the range of 0.5 $\mu m$ to 2.4 $\mu m$ or 6 $\mu m$ to 20 $\mu m$. Although carrier particles smaller than this range can be distinguished from blood cell components, it may be difficult for them to form pearl chains. For example, carrier particles with a size of 1 $\mu m$ or larger are preferred for efficient pearl chain formation. Therefore, carrier particles that yield agglutinates smaller than blood cell components preferably have a particle size of 1 $\mu m$ to 2 $\mu m$. For example, carrier particles with a particle diameter of 1 $\mu m$ to 1.8 $\mu m$ are preferred in the present invention. Most preferred particles are of 1.4 $\mu m$ to 1.8 $\mu m$. This size results in efficient pearl chain formation. When carrier particles with a particle size in this range are used, agglutinates composed of two to eight carrier particles can be clearly distinguished from blood cell components. Smaller carrier particles can also be used when oval shaped particles are used, which have a large dielectric polarization. The relationship among the number of carrier particles (1 $\mu m$ to 2 $\mu m$), the volume of agglutinates, and the difference from the volume of blood cell components is summarized below:

| number of carrier particles | volume of agglutinates | difference from the volume of blood cell components |
| --- | --- | --- |
| 2 | 1.05 $\mu m^3$ to 8.38 $\mu m^3$ | 79% to 97% |
| 5 | 2.62 $\mu m^3$ to 20.93 $\mu m^3$ | 48% to 93% |
| 8 | 4.19 $\mu m^3$ to 33.49 $\mu m^3$ | 16% to 90% |

On the other hand, carrier particles whose size largely exceeds 20 $\mu m$ may reduce the dispersibility or preservation stability of reagents in some cases. Therefore, they may require homogenization process prior to use. In addition, agglutinates formed with large carrier particles tend to be easily disrupted by dilution or movement of the carrier particles. The use of binding enhancers described later is useful for preventing such disruption.

The preferred average carrier particle diameter in immunoassays using pearl chain formation has been generally believed to be 0.5 $\mu m$ to 20 $\mu m$ when using, for example, latex particles. In order to enable measurement of samples containing blood cell components, the present inventors found additional conditions that can give agglutinates distinguishable from blood cell components and also maintain the benefits of immunoassays using pearl chain formation, and thereby completed the present invention.

In contrast to the 0.05- to 0.6-$\mu m$ carrier particles used in the conventional methods of latex agglutination turbidimetry, 1-$\mu m$ or larger particles are preferably used in the methods of the present invention. Agglutination reaction is accelerated by using the step of applying voltage pulses. As a result, agglutination reaction proceeds adequately in a short time even when larger particles are used. Larger carrier particles have the benefits described below. First, apertures with a larger diameter size can be used for particle measurement and as a result, apertures are hardly clogged. In addition, larger carrier particles can be easily distinguished from the measurement-interfering substances in body fluids. Measurement accuracy is improved as a result. These benefits of immunoassays using pearl chain formation are retained in the present invention.

A binding partner can be linked to particle carriers by methods suitable for the material. Those skilled in the art can appropriately select a method for linking the two. For example, latex particles can physically adsorb a protein such as an antigen, an antibody, or a fragment thereof. When latex particles have a functional group on their surface, a substituent that can be covalently linked to the functional group may be linked chemically. For example, -NH$_2$ in a protein can be linked to latex having -COOH.

Carrier particles bound to a binding partner may be subjected to blocking treatment, if required. Specifically, the binding of non-specific proteins onto the surface of carrier particles can be prevented by treating the surface of carrier particles with an inactive protein. Bovine serum albumin, skimmed milk, or such can be used as an inactive protein. Furthermore, detergents or sugars may be added to the dispersion medium to improve the dispersibility of carrier particles. Alternatively, antimicrobial agents may be added to particle carriers to prevent the growth of microorganisms.

The present invention comprises the step of applying voltage pulses to a reaction solution containing an affinity substance and carrier particles. A method in which carrier particles are aligned in an electric field to perform an agglutination reaction is known (JP-A No. H7-83928). Specifically, carrier particles can be aligned along an electric field by applying voltage pulses to a reaction solution containing an affinity substance and carrier particles.

When the principle of agglutination inhibition reaction is applied, an affinity substance and carrier particles are aligned in the presence of an agglutination reagent. The agglutination reagent can be contacted after carrier particles have been contacted with a target affinity substance. Alternatively, these three components can be contacted simultaneously by adding carrier particles to a premixture containing a target affinity substance and an agglutination reagent.

An alternating current component or a direct current component can be used for the voltage pulse, and these two may be combined at one's choice. An alternating voltage is preferable in that it allows reaction solutions to undergo electrolysis easily. For an alternating voltage, square waves, rectangular waves, sine waves, or such can be used. The power supply frequency for an alternating voltage can be adjusted arbitrarily depending on the ionic strength of the reaction solution (reagent). An alternating voltage is applied to provide an electrolytic intensity of 10-50 V/mm at its peak wave value. When the electrolytic intensity is less than 5 V/mm, carriers can hardly form pearl chains and as a result, the acceleration of agglutination reaction becomes inadequate. When the electrolytic intensity is greater than 50 V/mm, reaction solutions readily undergo electrolysis, sometimes making it difficult to measure agglutination reactions. More preferably, voltage is applied to provide an electric field intensity of 15 to 30 V/mm.

In common immunoassays that utilize pearl chain formation, a frequency of 50 KHz to 1 MHz is usually preferred. However, in the methods of the present invention in which blood cell components coexist, low frequencies may cause disruption of blood cell components. This is because low frequencies allow electric currents to easily pass through the reaction solutions. Therefore, in the present invention, for example, a frequency of 100 KHz to 10 MHz, or preferably 400 KHz to 1 MHz can be used.

Herein, the voltage pulse typically refers to a voltage having a wave or waveform in which the voltage level undergoes transitions from a steady state to a particular level, maintains the level for a finite time, and then returns to the original state. Alternating voltage is representative of such a voltage pulse. Alternating voltage is a periodic function of time with an average voltage value of zero. Alternating voltages include sine wave, rectangular wave, square wave, and sawtooth wave voltages, which have obvious periodic amplitudes. In general, the positive electric potential and the negative electric potential in an arbitrary cycle of alternating voltage have equal areas, making the sum of the two zero. Each area is defined by the curve above or below the horizontal axis, where the electric potential difference is zero. In the present invention, voltage pulses are applied to prevent electrolysis of reaction solutions. Accordingly, when electrolysis does not take place in a reaction solution, or if the electrophoresis, when it actually occurs, can be suppressed to an extent that does not substantially interfere with the reaction, voltage pulses having a non-zero sum of positive and negative electric potentials may be applied.

Herein, the square wave or rectangular wave voltage pulse refers to a power supply that comprises cycles of positive electric potential/zero electric potential difference/negative electric potential and a constant voltage for at least either the positive or negative electric potential. The interval between a state of zero electric potential difference and the succeeding zero state in square waves or rectangular waves is referred to as pulse width. Square wave refers to voltage pulses that form a nearly tetragonal shape when its voltage changes are drafted in a graph that has voltage on the vertical axis and time on the horizontal axis. The term "tetragonal" includes squares and rectangles. In contrast, rectangular waves are voltage pulses that have a rectangular shape, which does not include squares. Thus, square waves include rectangular waves. In the present invention, a generally preferred pulse width is 50 μsec or less, for example, in the range of 0.1 to 10 μsec.

There are no limitations on the duration of zero electric potential difference in square waves or rectangular waves. In general, the electric potential difference is zero at the moment of transition between positive and negative electric potentials. However, voltage pulses that maintain zero electric potential difference for a longer period may also be used in the present invention. For example, cycles of positive/negative electric potentials having a pulse width of 0.1 to 10 μsec may comprise a condition of zero electric potential difference that lasts 0.1 to 100 μsec.

In the present invention, there is no limitation on the temperature of the reaction solution when applying voltage pulses. The temperature can usually be 0°C to 20°C, for example, 0°C to 15°C, preferably 1°C to 8°C, or 2°C to 4°C. The temperature of the reaction solution will increase by the application of voltage pulses. To maintain the temperature of the reaction solution low, therefore, a cooling means may be advantageously utilized. Suitable cooling means for creating a local low-temperature environment include the Peltier element, for example. The Peltier element is an electronic element composed of a semiconductor that utilizes the Peltier effect discovered by Jean Charles A. Peltier. When a direct current flows through an N-type semiconductor and a P-type semiconductor, temperature will be absorbed at one of the semiconductors while heat radiation will occur at the other semiconductor (heat exchange phenomenon). The temperature at the side of heat absorption drops, resulting in cooling. Commercially available Peltier elements are generally capable of cooling down to around -10°C. The cooling capacity of the Peltier element can be freely controlled by the electric current supplied to semiconductors. Therefore, during the time when voltage pulses are applied, the temperature may be monitored by a temperature sensor and a Peltier element may be operated as necessary to maintain the temperature of a reaction solution within the predetermined range.

Alternatively, if the reaction solution is sufficiently cooled at the time of voltage pulse application and the temperature of the reaction solution is still within the predetermined range after the application of voltage pulses, cooling at the time of voltage pulse application may not always be necessary. For example, if the temperature of the reaction solution at the end of voltage pulse application is 20°C or lower, the temperature requirement can be satisfied without cooling during application. Positive cooling during voltage pulse application is not mandatory when the reaction solution is sufficiently cooled beforehand, and in addition, a rise in the temperature of the environment where the reaction solution is placed may be repressed.

In the present invention, the reaction solution to which voltage pulses are applied can be incubated in advance.

The conditions for incubation will be described later. When the reaction solution is incubated at as high a temperature as 37°C to 90°C, the solution is sufficiently cooled prior to applying voltage pulses. Normally, since the volume of the reaction solution is 1 mL or less, the reaction solution can be cooled in a very short time. Conditions in which a reaction solution that has been incubated at a high temperature is cooled and then subjected to voltage pulse application at 0°C to 20°C are preferred in the present invention.

The mechanism in which a rise in temperature during voltage pulse application affects the agglutination reaction in an inhibitory manner may be considered as follows. In a reaction solution to which voltage pulses are applied, alignment and dispersion of carrier particles repeatedly occur. Dispersion of carrier particles is effective for increasing the chance of a binding partner on carrier particles making contact with an affinity substance (or an agglutination reagent component) in a reaction solution. At the same time, alignment of carrier particles is effective for crosslinking multiple carrier particles and forming agglutinates by binding to an affinity substance (or an agglutination reagent component). When the motion of carrier particles in a reaction solution is intensive, the carrier particles however may not be sufficiently aligned. A condition under which the temperature of a reaction solution has increased may be considered as a condition under which the Brownian motion of carrier particles contained in the reaction solution becomes intensive, so that alignment of the carrier particles at the time of voltage application becomes difficult. As a result, alignment of carrier particles by voltage application is inhibited, and the agglutination reaction is inhibited. When the temperature of a reaction solution is controlled at the time of voltage application according to the present invention, sufficient effects of carrier particle alignment can be obtained by voltage application, so that inhibition of agglutination reaction as a result of temperature rise may be repressed.

It is also effective to increase the viscosity of reaction solution to suppress the movement of carrier particles in the reaction solution to which voltage pulses are applied. The viscosity of reaction solution in common agglutination reactions is less than 0.75 mPas (Pascal second, or poise (P)). With such viscosity, the motion of carrier particles may not be repressed, and the agglutination reaction may be inhibited. On the contrary, the present inventors confirmed that, at a viscosity of 0.8 mPas or higher, the agglutination reaction may proceed efficiently. More specifically, the present invention provides methods for agglutinating carrier particles, comprising the step of applying voltage pulses to a reaction solution, which contains a particular substance and carrier particles bound to a binding partner that has the activity to bind to the particular substance, wherein the viscosity of the reaction solution is maintained at 0.8 mPas or higher during voltage application. More specifically, the present invention provides methods for measuring an affinity substance, which comprises the aforementioned steps of (1) to (3) or (1') to (3'), in which the viscosity of reaction solution is 0.8 mPas or more.

In the present invention, the viscosity of the reaction solution is typically 0.8 mPas or higher, for example, from 1 to 3 mPas, and preferably from 1 to 2 mPas. The viscosity of the reaction solution can be adjusted by adding compounds that are capable of adjusting viscosity. As compounds capable of adjusting viscosity, any compound that does not interfere with the binding between an affinity compound and a binding partner may be utilized. For example, bovine serum albumin, casein, glycerin, sucrose, or choline chloride, may be added to increase the viscosity of a reaction solution. The amount of compound to be added may be appropriately selected, for example, from 0.05 to 5%, more preferably 0.1 to 3%, and even more preferably 0.3 to 1%. In addition, even if the composition of a reaction solution remains the same, the viscosity will generally increase when the temperature of the reaction solution lowers. Thus, application of voltage pulses under low-temperature condition is effective in terms of increasing the viscosity of a reaction solution.

Those skilled in the art can determine the appropriate amount to add, by adding these compounds to a reaction solution and then measuring its viscosity under the temperature conditions for voltage pulse application. Methods for determining liquid viscosity are known. In general, rotational viscometers, ultrasonic viscometers, oscillational viscometers, and such are used.

In the present invention, voltage pulses can be applied to a reaction solution from any direction. For example, voltage pulses can be applied from multiple different directions. Specifically, for example, two pairs of electrodes can be combined to apply voltage pulses to a reaction solution. Alternatively, voltage pulses in different directions can be applied by moving the electrodes with respect to the reaction solution. For example, by rotating the electrodes, voltage pulses can be applied at any angle. One pair or two or more pairs of electrodes can be moved.

In the present invention, the concentration of carrier particles in the reaction solution can be, in the case of latex particles, typically selected from the range of 0.1 w/v% to 1 w/v% and preferably from the range of 0.2 w/v% to 0.5 w/v%. The concentration of carrier particles in the reaction solution is adjusted depending on the proportion and concentration of a medium containing blood cell components used as a sample, reagent solution containing latex particles, and diluent and such added as necessary. For example, the carrier particle concentration of 0.1 w/v% to 1 w/v% is so high for common immunoassays that use the agglutination of carrier particles as an indicator. Such a high carrier particle concentration will cause non-specific agglutination and erroneous results due to unagglutinated particles being counted as agglutinates. However, in the present invention, agglutinated particles or unagglutinated particles are counted using their three-dimensional information as an indicator after the immunological reaction. Erroneous counting results can be prevented by using the three-dimensional information as an indicator. For example, the use of three-dimensional information as an indicator prevents merely overlapping carrier particles from being erroneously counted.

In the present invention, salts may be added to a reaction solution to accelerate agglutination reaction. For example, a relatively high (10 mM or higher) concentration of salt may be added to accelerate agglutination reaction. However, a salt concentration of 600 mM or higher in a reaction system is unfavorable because such a higher concentration promotes electrolysis of the reaction solution. The salt concentration is more preferably in the range of 10 to 300 mM, most preferably in the range of 25 to 150 mM. When there is a possibility that a biological sample itself might contain a salt that accelerates agglutination reaction, the reagent's salt concentration may be adjusted so that the final salt concentration in a reaction solution falls within the range shown above. When direct-current voltage pulses are used, electrolysis takes place in a reaction solution even at a salt concentration of about 6 mM. Therefore, it is difficult to measure the biologically specific agglutination reaction in the presence of a salt.

Salts of the present invention can be selected from those that accelerate biologically specific agglutination reactions. Such salts include but are not limited to, for example, sodium chloride, potassium chloride, sodium nitrate, potassium nitrate, and ammonium chloride. A preferred salt of the present invention gives 100 $cm^2/(\Omega \cdot mol)$ or higher molar electric conductivity in a 10mM aqueous solution at 25°C. More specifically, such preferred salts include, for example, sodium chloride, potassium chloride, and ammonium chloride.

In the present invention, a medium comprising blood cell components may be an undiluted solution or may be automatically diluted for use in measurement. The dilution ratio can be set arbitrarily. When multiple types of reagents are necessary for the reaction, the reagents can be sequentially added. Examples of reagents constituting the multiple reagents mentioned herein include the following reagents.

In the present invention, it is possible to use reagents for degrading and/or absorbing in advance substances that cause non-specific reactions. Such reagents are useful as reagents containing non-specificity inhibitors. Reagents containing non-specificity inhibitors are combined with reagents containing carrier particles to constitute multiple reagents. For example, reagents containing non-specificity inhibitors can be combined in advance with samples. For example, conventionally known non-specificity inhibitors may be used.

It has been demonstrated that various substances that cause non-specific reactions are included in samples in immunoassays. For example, globulins such as rheumatoid factors may interfere with immunological reactions involved in immunoassays. Non-specificity inhibitors are used to prevent globulins from interfering immunoassays. For example, antibodies that recognize globulins can absorb the non-specific actions. Rheumatoid factors are globulins derived from IgG or IgM. Therefore, rheumatoid factors can be absorbed using anti-human IgG antibodies or anti-human IgM antibodies. Methods for preventing interference by degrading non-specificity causative substances are also known. Specifically, globulins can be degraded by reduction, thereby suppressing their interference activity. Globulins are reduced by dithiothreitol, 2-mercaptoethanol, or such.

Furthermore, two or more reagents containing carrier particles bound to binding partners having different binding activities can be combined. Such a composition will allow simultaneous measurement of different types of target affinity substances. Each reagent can be individually added. Alternatively, multiple reagents can be combined in advance and then mixed with a sample.

Samples and reagents are preferably mixed in advance before voltage application. They can be physically mixed using a stirring bar. Alternatively they can be mixed by electrical methods. An example of electrical methods is a method of physically moving the position of carrier particles by intermittently applying voltage pulses from different directions.

In the present invention, it is possible to incubate in advance a reaction solution in which a target affinity substance is mixed with carrier particles coupled to a binding partner having an activity to bind to the target affinity substance. The present inventors have shown that formation of agglutinates after voltage pulse application is promoted by incubation of the reaction solution prior to voltage pulse application. That is, incubation before voltage pulse application promotes the reaction.

Incubation of a reaction solution is carried out, for example, at a temperature above room temperature. It is desirable that the incubation temperature is as high as possible as long as activities of the various reactive components contained in the reaction solution can be maintained. The incubation time is not limited. Namely, incubation can be carried out at an incubation temperature that does not cause modifications to the reactive components. The longer the incubation time, the further the acceleration effect will be enhanced. It is therefore desirable to preliminarily set the conditions of temperature and time in which the necessary level of acceleration effect can be expected.

Specifically, examples of the temperature condition for incubation are typically 37 to 90°C, preferably 40 to 90°C or 45 to 80°C. For example, a protein antigen as an affinity substance can be measured using an antibody as a binding partner according to the present invention. Antibody- or antigen-composing proteins are known to denature at high temperature. For example, many proteins do not denature at incubation conditions of 30 minutes at 56°C, which are conditions generally used for inactivating serum. In addition, the inventors have observed that protein denaturation is negligible even at a temperature as high as 90°C in conditions of low protein concentrations such as in an immunoassay if the treatment is for a short period of time. For example, it has been observed that incubation for five to 180 seconds in the range of 45 to 80°C yields approximately the same level of reaction-accelerating effect. Therefore, preferred incubation conditions in the present invention are preferably 45°C to 80°C, or more preferably 50°C to 65°C, for 5 seconds or longer, for example, 5 to 30 seconds.

In the present invention, samples containing blood cell components are incubated at a high temperature. According to the findings by the present inventors, effects of electric field, such as disruption of blood cell components, have not been observed under conditions of, for example, 60°C for 90 seconds, 80°C for 30 seconds, or such.

In the present invention, it is needless to say that the present invention also includes longer incubation time. When reduction of incubation time is desired, a short period of five seconds or more may be adopted and the desired reaction-accelerating effect can be expected without sacrificing the reaction time. For example, under temperature conditions exceeding 80°C, disruption of blood cells and protein denaturation can be avoided by setting the incubation time at 5 to 30 seconds.

Normally, the size of blood cells is within a certain range. Therefore, when counting particles, signals derived from blood cells can be specifically detected. However, if blood cells are disrupted, components of various sizes are produced. Since components produced by the disruption are not uniformly sized, it is difficult to specifically determine which signals are derived from blood cells. In other words, the disruption of blood cells may cause noise. Therefore, effects of noise caused by components of disrupted blood cells can be prevented by avoiding the disruption of blood cells. In particular, the provision of low noise level conditions is of great significance when highly sensitive measurements are required.

Alternatively, high-temperature conditions will not become a problem when the method of the present invention is applied to reactions of heat-resistant substances. DNA, for example, is highly stable under high-temperature conditions. In cases where binding between DNAs is sought to be measured based on agglutination of carrier particles, higher temperatures may be chosen for the incubation temperature.

In the present invention, the mechanism in which a reaction is accelerated by incubation can be explained as follows. Carrier particles that align as a result of voltage pulse application form agglutinates through crosslinking between binding partners immobilized thereon and binding partners on other carrier particles via affinity substances. The series of reactions are thought to take place when the carrier particles become aligned. The findings attained by the inventors confirmed that incubation prior to application of voltage pulses contributes to the reaction efficiency. It was also revealed that, during such incubations, the binding partner on carrier particles becomes bound to the affinity substance. In fact, it is believed that the reaction can be made more efficient by allowing the binding partner to capture the affinity substance prior to alignment of carrier particles by applying voltage pulses.

Carrier particles are supposed to have a far more degree of freedom under conditions in which voltage pulses are not applied, as compared with when voltage pulses are applied. A binding partner on a carrier particle is therefore capable of making contact with an affinity substance such that the binding partner can capture the affinity substance. Upon application of voltage pulses, carrier particles with a binding partner that has captured the affinity substance will be aligned across the electric field along with other carrier particles. Since the binding partner has already captured the affinity substance, agglutinates are quickly formed via binding with the binding partner of other nearby carrier particles. When an electric field is intermittently applied, chances of making contact will increase through repetition of alignment and dispersion of carrier particles, and formation of agglutinates will be accelerated.

In methods of detecting agglutination of carrier particles following the application of voltage pulses and measuring affinity substance by using the agglutination as an indicator, agglutinates of carrier particles can be said to form via the following primary and secondary reactions.

Primary reaction: A reaction in which a binding partner on carrier particle captures an affinity substance. The crosslink structure between carrier particles via the affinity substance does not necessarily have to be formed yet.

Secondary reaction: A reaction in which the binding partner on multiple carrier particles binds to an affinity substance. As a result, a crosslink structure (that is, agglutinate) is formed between carrier particles via the affinity substance.

The secondary reaction is accelerated by application of voltage pulses. Up until now, however, no specific conditions for accelerating the primary reaction have been revealed. The present invention can therefore be considered to provide conditions for accelerating the primary reaction. Namely, the incubation step before application of voltage pulses in the present invention can be said to be a step for accelerating the primary reaction.

In the present invention, water-soluble polymers can be added to a reaction solution before application of voltage pulses. By detecting the binding between an affinity substance and a binding partner based on particle agglutination reaction in the presence of a water-soluble polymer, enhancement or stabilization of the agglutination reaction can be achieved. The concentration of water-soluble polymer in a reaction solution may be appropriately selected from, for example, 0.05 to 5%. The concentration is more preferably 0.1 to 3%, and even more preferably 0.3 to 1%. The possibility of nonspecific agglutination reaction tends to increase for compounds with high agglutination reaction-enhancing ability, at a concentration exceeding 5%. Also, at 0.05% concentration or lower, sufficient efficiency may not be expected.

As water-soluble polymers, polyethylene glycol, dextran, carboxymethylcellulose and such may be used. The molecular weight of polyethylene glycol is preferably 6,000 to 2,000,000. These water-soluble polymers may be used alone or in combination of two or more. To add a water-soluble polymer to a reaction solution, the required amount may be added beforehand to a carrier particle-containing reagent. Alternatively, the water-soluble polymer may be mixed as a reagent different from the carrier particle reagent. For example, the water-soluble polymer may be added to diluted sample solutions. It may also be added to multiple reagents to be mixed and diluents.

Preferably, a two- reagent system is used, where a water-soluble polymer is contained in a first reagent such as a buffer solution, and then mixed with a second reagent containing carriers before use in measurement. In this case, nonspecific absorbents that absorb nonspecific substances such as heterophile antibodies, or substances that absorb rheumatoid factors may be added to the first reagent.

The improvement of reaction efficiency due to incubation prior to voltage pulse application based on the present invention can also be applied to agglutination inhibitory reactions. Specifically, a reaction solution containing a target affinity substance can be incubated before or after combining it with agglutination reagent components in the methods of the present invention comprising the aforementioned steps (1') to (3'). The incubation conditions are the same as the conditions described above. Furthermore, water-soluble polymers can also be added to the reaction solution in agglutination inhibitory reaction systems.

As described above, incubation of the reaction solution before voltage pulse application is effective for promoting aggregation reactions of carrier particles. Here, a higher incubation temperature is more effective as already discussed above. On the other hand, the temperature of the reaction solution subjected to voltage pulse application increases due to Joule heat. The Joule heat is generated in a conductor when an electric current passes through. The present inventors discovered that while high temperature conditions during the incubation act on the agglutination reaction in a promotional manner, increased temperature during voltage pulse application acts on the agglutination reaction in an inhibitory manner. Therefore, it is advantageous to keep the temperature of the reaction solution low during voltage application.

The present invention provides methods for measuring an affinity substance, which comprise the steps of:

(1) applying a voltage pulse to a reaction solution in which a target affinity substance is mixed with carrier particles that are bound to a binding partner having an activity to bind to the target affinity substance;
(2) counting both or either of agglutinates of carrier particles formed due to binding of the target affinity substance, and/or unagglutinated carrier particles which are not bound to the target affinity substance, by using their three-dimensional information as an indicator; and
(3) determining the level of the target substance based on both or either of the level of agglutinate formation and/or the level of unagglutinated carrier particles,

wherein the target affinity substance is included in a medium comprising blood cell components;
wherein step (1) is carried out in the presence of said medium; and wherein carrier particles in step (1) are of a size that yields agglutinates having a volume that can be distinguished from blood cell components:
Each step constituting the measurement methods of the present invention are specifically described below.

The mixed reaction solution is then transferred to a chamber equipped with electrodes, and voltage pulses are applied. When an electric field is applied, the carrier particles develop a dielectric polarization and electrostatically attracted to each other to align linearly. This phenomenon is called pearl chain formation. The linearly aligned carrier particles redisperse immediately after the electric field is terminated. However, if a biologically specific reactive substance is present during pearl chain formation, carrier particles involved in the biologically specific reaction remain in the form of agglutinates and do not disperse even after termination of the electric field. The presence of the biologically specific reactive substance can be detected or measured by measuring these agglutinated particles involved in the biologically specific agglutination reaction and/or the dispersed carrier particles not involved in the reaction.

In the measurement methods of the present invention, agglutinates of carrier particles formed due to binding of a target affinity substance, and/or unagglutinated carrier particles that are not bound to the target affinity substance are counted using their three dimensional information as an indicator. In the present invention, the particles can be measured after the electric field is terminated. Alternatively, the particles placed in an electric field can be measured without terminating the electric field. For example, the particles placed in an electric field can be counted by taking them out from the electric field. Furthermore, the step of dispersing the particles can be conducted before counting the particles. The dispersion step before counting can disperse particles that have agglutinated due to nonspecific factors. As a result, the accuracy of measurement is expected to be improved. The particles can be dispersed by stirring or diluting the reaction solution.

When the reaction solution is diluted in the present invention, the binding between the affinity substance and the binding partner, or between the agglutination reagent and the binding partner can be strengthened before the aforementioned step (3) or (3'). The strengthened binding can prevent disintegration of the agglutinates resulting from dilution. For example, the method of diluting the reaction solution under conditions in which voltage pulses are applied is a preferred dilution method in the present invention. More specifically, dilution is carried out by adding the reaction solution to a diluent to which voltage pulses are applied. The diluent is placed between electrodes and subjected to voltage pulse application. In other words, the diluent solution is placed between electrodes opposed to each other.

The reaction solution can be diluted under conditions in which voltage pulses are applied. In this case, the size and interval of the electrodes are not particularly limited. Specifically, the initial contact between the diluent and the reaction solution after pearl chain formation is carried out in an electric field across opposing electrodes.

In the dilution step, voltage pulses are preferably those of an alternating voltage. Conditions for voltage pulse application can be any conditions that do not induce electrolysis of the reaction solution or the diluent. Voltage of the voltage pulses is, for example, 0.1 V to 1.2 V, and more preferably 0.3 to 0.9 V. Frequency of the voltage pulses is, for example, 100 KHz to 10 MHz, and more preferably 400 KHz to 1 MHz. The voltage pulses may assume any waveforms. Specifically, the voltage pulses may appear as square waves, sine waves, triangular waves, etc. More preferably, the voltage pulses are in square waves. In the present invention, it is desirable to apply voltage pulses during times including at least moments of contact between the reaction solution and the diluent. Effects of enhancing the binding in dilution can be expected even with only a very short period of application time. More specifically, the application time is 0.5 to 30 seconds, and typically one to ten seconds, for example, one to five seconds.

As a diluent, a salt-containing solution may be used. Specifically, a physiological saline solution, glycine buffer, phosphate buffer, or such to which a salt of 50 mM to 600 mM has been added may be used. Salts include sodium chloride, potassium chloride, and calcium chloride. Diluents may contain preservatives, such as sodium azide, surface active agents, such as Triton X-100, glycerin, sucrose, etc.

The binding reaction between an affinity substance (or an agglutination reagent) and a binding partner which form an agglutinate is enhanced by performing dilution while applying voltage pulses. The dipole moment effect of an electric field as a result of voltage pulses is thought to enhance the binding between them. In any case, it has been confirmed that dilution under the conditions of voltage pulse application represses disruption of agglutinates so that the dilution of a reaction solution may be achieved while maintaining the agglutinates.

Alternatively, as a dilution means capable of enhancing the binding between an affinity substance and a binding partner or the binding between an agglutination reagent and a binding partner, a binding enhancer capable of enhancing the binding between them may be utilized. Herein, a binding enhancer refers to a component capable of enhancing the binding between them when added to a reaction solution. For example, binding between proteins may be enhanced by adding compounds such as glutaraldehyde or carbodiimide. Immunological binding between a protein antigen and an antibody may therefore be enhanced by glutaraldehyde, carbodiimide, or such. These compounds are preferable binding enhancers.

A binding enhancer acts on the functional groups of an affinity substance and a binding partner to chemically bind the two. Alternatively, in an agglutination-inhibiting reaction system, it enhances the binding between an agglutination reagent and a binding partner. As a result, an agglutinate formed by the binding of the two attains high physical stability. For example, when the affinity substance or agglutination reagent and the binding partner are proteins, they have intramolecular amino or carboxyl groups. These functional groups are crosslinked by chemicals such as glutaraldehyde and carbodiimide.

The concentrations of a binding enhancer in a reaction solution may be appropriately set according to the type of binding enhancer. More specifically, in the case of glutaraldehyde, for example, the final concentration in a reaction solution is typically from 0.1 to 25%, and preferably from 0.2 to 18%. The binding enhancer may be added to a reaction solution containing a conjugate of affinity substance and binding partner before diluting the reaction solution. The reaction solution to which a binding enhancer has been added can be diluted after incubation at 37°C for several seconds to about 20 seconds, preferably two to ten seconds, or two to five seconds. When glutaraldehyde or carbodiimide is used as a binding enhancer, the reaction solution may be diluted immediately after the addition.

Addition of a binding enhancer is effective in the dilution step of the present invention. In the present invention, binding enhancers can be also combined with the aforementioned dilution step performed under conditions where voltage pulses are applied. Specifically, after adding a binding enhancer to the reaction solution, the reaction solution can be diluted under voltage-pulsed conditions according to the conditions mentioned above. Alternatively, the reaction solution can be diluted using a diluent supplemented with a binding enhancer under voltage-pulsed conditions according to the aforementioned conditions. By combining them, the binding enhancing activity is strengthened.

In the present invention, dilution of the reaction solution refers to decreasing the concentration of the carrier particles in the reaction solution by mixing the reaction solution with the diluent. The concentration of carrier particles in the reaction solution is determined by the amount of sample and the amount of carrier particles provided as a reagent. Furthermore, in the present invention, the concentration of carrier particles in the reaction solution is normally set within a range that can promote agglutination of carrier particles by pearl chain formation. Specifically, the concentration of carrier particles in the reaction solution is typically 0.1 to 1 % by weight, more preferably 0.2 to 0.5% by weight, as discussed above. This can be diluted, for example, 100 times or more, typically 1,000 times or more, specifically 1,000 to 100,000 times, or preferably 2,000 to 40,000 times. The diluted concentration of the carrier particles is $0.1 \times 10^{-5}$ to 0.001 % by weight and preferably $0.25 \times 10^{-5}$ to $5 \times 10^{-5}$ % by weight.

In the present invention, the particles are counted using their three-dimensional information as an indicator. In the present invention, the "counting using the three-dimensional information as an indicator" refers to determining the three dimensional information of the particles and/or agglutinates, and counting the particles and/or agglutinates based on those results. Known methods of analyzing microscope images determine the level of aggregation based on two-dimensional information. Therefore, the methods of the present invention which use three-dimensional information as an indicator are clearly distinguished from known methods.

There is no limitation on the method of measuring three-dimensional information. Herein, "counting" refers to determining the number of particles and/or agglutinates. The number of particles and/or agglutinates can be determined by simple counting. Alternatively, agglutinated particles and unagglutinated particles can be counted separately. Furthermore, in measuring agglutinated particles, the number of agglutinates may be determined for each number of agglutinated particles. There are known methods for counting particles using three-dimensional information as an indicator.

Measurement methods that are based on physical principles can be advantageously applied as the particle-counting methods in the present invention. Herein, "physical ' measurement methods" refers to measurement methods that enable the evaluation of inherent physical information of particles or agglutinates. In other words, the inherent physical information of particles or agglutinates is a result of true measurement. On the other hand, methods that analyze two-dimensional information obtained from graphic information also detect non-agglutinated overlapping particles as agglutinates. Such detection results are not considered inherent physical information of particles.

The use of a flow system is advantageous when the particles or agglutinates are measured physically. A flow system is a system which is capable of analyzing physical information of particles that pass through a minute flow cell. Physical measurements can be achieved conveniently by using a flow system. Specifically, physical measurements in the present invention comprise the step of counting by using a flow system to measure the three-dimensional information of particles and/or agglutinates. Methods that use three-dimensional information as an indicator to physically count particles include, for example, the Coulter principle and laser diffraction/scattering methods.

The Coulter principle (USPA 2656508 in 1953) is an analysis method for determining the volume of a particle based on the change of electric resistance resulting from passing of the particle through an aperture (small hole), which has electrodes on both sides. When a minute electric current is allowed to pass through an electrolytic solution between two electrodes, particles that are suspended in the electrolytic solution are aspirated, passed through an aperture, and then replaced by an equivalent volume of electrolytic solution. As a result, the electric resistance between electrodes is altered. The particle number and size (volume) can be determined by measuring this change. The electrostatic capacity method is available as a method for measuring volume; however, most of the methods that are in practical use are electric resistance methods.

The aperture size can be appropriately adjusted to accommodate the subject particle of analysis. In general, it is advantageous to have an aperture size that is several to several hundred times greater, for example, several to a hundred times greater, preferably 5 to 50 times greater than the mean particle diameter of carrier particles. In this case, highly accurate and sensitive measurements can be realized by detection of signals proportional to the volume. The sensitivity is higher when the aperture size-to-particle diameter ratio is small. However, when the ratio is too small, particles tend to clog up the aperture; when the ratio is too large, sensitivity of particle detection decreases; both cases are unfavorable.

More specifically, when carrier particles with a diameter of, for example, 1 $\mu$m to 2.4 $\mu$m, and especially 1 $\mu$m to 1.8 $\mu$m, are counted, the aperture size can be selected from the range of 30 $\mu$m to 100 $\mu$m, preferably 40 $\mu$m to 80 $\mu$m, for example, 50 $\mu$m to 75 $\mu$m. The carrier particle size of 2 $\mu$m to 3 $\mu$m is a particularly preferred in the methods for measuring affinity substances according to the present invention. Thus, the present invention provides methods for measuring an affinity substance, which comprise the following steps of (1) to (3) or (1') to (3'), wherein the target affinity substance is included in a medium comprising blood cell components, and wherein step (1) or (1') is carried out in the presence of said medium:

(1) applying a voltage pulse to a reaction solution in which a target affinity substance is mixed with carrier particles with an average particle diameter of 1 $\mu$m to 1.8 $\mu$m that are bound to a binding partner having an activity to bind to the target affinity substance;

(2) counting both or either of agglutinates of carrier particles formed due to binding of the target affinity substance, and/or unagglutinated carrier particles which are not bound to the target affinity substance, by using their three-dimensional information as an indicator according to the Coulter principle with an aperture size of 50 $\mu$m to 75 $\mu$m; and

(3) determining the level of the target substance based on the level of agglutinate formation and/or the level of unagglutinated carrier particles; or

(1') applying a voltage pulse to a reaction solution in which an agglutination reagent component is mixed with a target affinity substance and carrier particles with an average particle diameter of 1 $\mu$m to 1.8 $\mu$m which are bound to a binding partner having an activity to bind to the target affinity substance, wherein the carrier particles agglutinate due to the agglutination reagent, and the agglutination is inhibited by the target affinity substance;
(2') counting both or either of agglutinates of carrier particles formed due to binding of the agglutination reagent, and/or carrier particles whose agglutination is inhibited by binding of the target affinity substance, based on their three-dimensional information as an indicator according to the Coulter principle with an aperture size of 50 to 75 μm; and
(3') determining the level of the target substance based on either or both of the level of agglutinate formation and/or the level of unagglutinated carrier particles.

In general, the smaller the aperture size, the more accurately unagglutinated particles can be counted. Conversely, greater aperture size reduces the chance of an aperture being clogged with agglutinated particles. Aperture clogging decreases analysis efficiency, which can be improved by reducing the clogging frequency. For example, if agglutinated particles are predicted to be formed in great numbers, aperture clogging can be prevented by setting the aperture size to be slightly larger. Alternatively, a similar effect can be expected by using carrier particles with a small particle diameter. Further, the proportion of agglutinated particles may be reduced by diluting the sample to thereby prevent aperture clogging. In general, appropriate conditions may be selected for each case depending on the expected detection sensitivity, the predicted concentration of target substance to be detected, and the device configuration (aperture size, in particular).

The proportion of agglutinated particles can be determined by counting agglutinated particles by the procedure described above. The "proportion of agglutinated particles" refers to the proportion of agglutinated particles among the total particles counted. The proportion of agglutinated particles is also referred to as "percent agglutination (agglutination rate)". Furthermore, percent agglutination is determined for standard samples with known analyte concentrations, and the relation between the two is plotted on a graph to produce a standard curve. The concentration of a target affinity substance in a sample can be revealed by checking percent agglutination of the sample against the graph.

Alternatively, the above-mentioned standard curve can also be expressed as a regression equation. Once a regression equation is obtained, the concentration of a target affinity substance can be calculated by substituting percent agglutination into the regression equation.

On the other hand, laser diffraction/scattering methods are used to count particles and measure their mean diameter by detecting fluctuations generated from laser irradiation of particles. In either case, for the purpose of improving measurement accuracy, it is preferable to dilute reaction particles, apply sonication, and/or use a sheath flow system, and such to prevent false measurements of particles.

Methods for measuring particle volume also include the methods described below.

Centrifugal sedimentation method: a method for determining particle diameter distribution by the Stokes equation, which represents the relation between particle sedimentation rate in a solution and particle diameter. Photocentrifugal sedimentation methods use a phenomenon based on Stokes' law: larger particles sediment faster than smaller ones when they have the same specific gravity. The particle concentration is analyzed as the change in turbidity from light transmission. The particle size distribution can be determined by the procedure described above.

Capillary system: Hagen-Poiseuille flow is generated in a capillary when the viscous fluid that flows through the capillary has a low Reynolds number. Since this flow is faster at the center of the capillary and slower near the capillary wall, large particles travel in fluxes that are faster on average and smaller particles travel in fluxes that are slower on average. Briefly, particles traveling through a capillary of given length are size-separated and detected according to the differences of their moving velocities.

Three-dimensional image analysis: Three-dimensional particle information can be obtained by analyzing graphic information of two or more images taken from different angles. Alternatively, three-dimensional particle information can be obtained by scanning graphic information along the z axis in the xy plane.

In the measurement methods of the present invention, agglutinated (or unagglutinated) carrier particles are counted using the three-dimensional information. The target affinity substance of the measurement is measured qualitatively or quantitatively based on the counting results. In such qualitative measurements, the presence of a target affinity substance is indicated by the presence of agglutinated particles. Alternatively, detection of agglutination inhibition in an agglutination inhibition reaction proves the presence of the target of measurement.

Alternatively, in such quantitative measurements, the level of agglutination can be correlated with the amount of target affinity substance. More specifically, samples containing a known concentration of affinity substance are measured preliminarily using the measurement methods of the present invention to unravel the relation between the amount of affinity substance and the result of agglutinated particle detection based on the three-dimensional information. Then, samples are measured by the same measurement procedure. The amount of affinity substance can be determined from the result of agglutinated particle detection based on volume. In the case of an agglutination inhibition reaction, quantitative measurements can also be achieved by the same procedure described above.

In methods for counting particles and/or agglutinates, formulae such as [number of particles that form agglutinates of two or more particles]/[total number of particles], or [number of single particles]/[total number of particles], can be selected as means for counting a specific number of particles according to the purpose. The total number of particles may be determined as the total number of particles measured within a fixed time period of measurement, or in a literal sense, the total number of particles in a reaction solution when the entire reaction solution is the target of analysis. When the total volume of a reaction solution is known, the total number of particles in a reaction solution can be estimated by counting a portion of the reaction solution.

Furthermore, in the present invention, it is possible to determine the volume of blood cell components occupying a sample medium containing blood cell components. In the present invention, both agglutinated and unagglutinated carrier particles are distinguished from blood cell components. As a result, blood cell components can be counted separately from these carrier particles. By counting the blood cell components using their three-dimensional information as an indicator, it is also possible to determine their volume. The proportion of blood cell components to the sample medium can also be determined from the determined volume of blood cell components. The proportion of blood cell components to whole blood components thus determined has nearly the same meaning as a hematocrit value.

Therefore, for example, when whole blood is used as a sample, the volume obtained by subtracting the volume of blood cell components from the volume of whole blood corresponds to the volume of serum (or plasma). Based on the ratio between both volumes, the concentration of the target substance included in the whole blood that has been measured according to the present invention can be corrected to a serum concentration.

For example, since the detection method using the Coulter principle (USPA 2656508, 1953) can determine the number and size (volume) of particles, it can simultaneously determine the size distribution of unagglutinated and agglutinated particles and the volume and number of blood cell components. That is, since the volume and number of blood cell components in the reaction solution can be determined, the total volume of blood cell components can be calculated. Therefore, the volume percentage of blood cell components in a whole blood sample used for the measurement can be determined. Since plasma (or serum) samples are obtained by removing blood cell components from whole blood, measured values can be converted into plasma (or serum) levels by correction with the percentage of blood cell components.

$$\text{AR (plasma)} = \text{AR (whole blood)} \times 100 / (100 - \text{blood cell components \%})$$

In the present invention, the Coulter principle can be used to determine the volume of blood cell components. For example, for counting erythrocytes, the number and volume of red blood cells can be determined using a Coulter counter with an aperture size of 200 $\mu$m or less. Preferred aperture size for determining blood cell volume is 30 $\mu$m to 200 $\mu$m.

Alternatively, the affinity substance can be detected or measured based on the number of particles and/or agglutinates detected during a certain period of time by an electric resistance method, laser diffraction/scattering method, or such. That is, the number of particles counted decreases with time because single particles agglutinate to form agglutinates in agglutination reactions. Alternatively, it is possible to use the time required for counting a specific number of particles and/or agglutinates as an indicator. When such counting methods are used in the present invention, the relation between the number of particles and/or agglutinates and the amount of affinity substance can be expressed in a regression equation.

For particles that have been sensitized with an antibody, the proportion of agglutinates comprising two or more particles increases depending on the antigen concentration. In this case, percent agglutination represented by [number of particles forming agglutinates consisted of two or more particles]/[total number of particles] converges to 1.00 (100%).

When compared with methods that analyze two-dimensional graphic data, methods that measure three-dimensional particle information, whether it be the Coulter principle or a laser diffraction/scattering method, allow high-accuracy analyses even with a simple device configuration. As described above, the volume of reaction solution is restricted in analyses of two-dimensional graphic data. In contrast, there are no limitations on the reaction solution volume in methods that measure three-dimensional information using flow-based analytical techniques. In addition, there are no limitations on the physical geometry of reaction space. These reasons attribute to a simpler device configuration. The fact that the reaction solution volume can be set freely further contributes to the reproducibility and detection sensitivity.

Alternatively, the present invention provides methods for measuring an affinity substance, which comprise the steps of:

(1') applying a voltage pulse to a reaction solution in which an agglutination reagent component is mixed with is a target affinity substance and carrier particles that are bound to a binding partner having an activity to bind to the target affinity substance, wherein the carrier particles agglutinate by the agglutination reagent, and the agglutination is inhibited by the target affinity substance;

(2') counting both or either of agglutinates of carrier particles formed due to binding of the agglutination reagent, and/or carrier particles whose agglutination is inhibited by binding of the target affinity substance, based on their three-dimensional information as an indicator; and

(3') determining the level of the target substance based on either or both of the level of agglutinate formation and/or the level of unagglutinated carrier particles,

wherein the target affinity substance is included in a medium comprising blood cell components,

wherein step (1) or (1') is carried out in the presence of said medium, and the carrier particles in step (1) or (1') are of a size that yields agglutinates with a volume that can be distinguished from blood cell components.

The principle of immunological particle agglutination reaction methods based on agglutination inhibitory reactions that use agglutination reagents have been already described. The present invention can be applied to immunological particle agglutination reactions by the above-mentioned steps. Voltage pulse application and analysis of the level of agglutinate formation or the level of unagglutinated carrier particles can be carried out by the methods as specifically described above.

When conducting the present invention based on the principle of agglutination inhibiting reactions, it is preferred to select conditions so that more agglutinates are formed by two or more particles. Alternatively, methods for evaluating the aggregation level using "number of single particles / total number of particles" as an indicator are preferred. When the present invention is based on the principle of agglutination inhibiting reactions, the use of this arithmetic expression is expected to yield higher sensitivity than analyses based on the arithmetic expression of "number of particles forming agglutinates of two or more particles / total number of particles".

The methods of the present invention can be carried out using an apparatus equipped with a system for applying a voltage pulse while carrying a reaction solution, and a system for counting carrier particles. For example, an apparatus for measuring affinity substances comprising the following means can be used to perform the present invention:

(a) a space for carrying a target affinity substance and carrier particles bound to a binding partner having an activity to bind to the target affinity substance;

(b) electrodes for applying a voltage pulse to the carrier particles carried in said space; and

(c) means for counting both or either of agglutinates formed by agglutination of carrier particles and/or unagglutinated carrier particles carried in said space, based on their three-dimensional information as an indicator.

In the present invention, any space for holding the reaction solution can be used for (a) the space for carrying a target affinity substance and carrier particles bound to a binding partner having an activity to bind to the target affinity substance. A space with a small volume is advantageous for reacting a small amount of sample. For example, a space of about 1 μL to 10 mL or preferably 10 μL to 500 μL can be used. If necessary, this space can be equipped with a means to supply samples or reagents, or a means to measure carrier particles which will be described later.

Next, (b) electrodes for applying a voltage pulse to the carrier particles carried in said space in the present invention will be described. Electrodes for linearly aligning carrier particles in an electric field are described in, for example, the prior art documents described above. These known electrodes can be used in the present invention. The apparatus of the present invention can be equipped with a power source for supplying voltage to the electrodes.

Electrodes for applying voltage pulses in the apparatus for carrying out the present invention are composed of at least one pair of (two) electrodes. To give multiple voltage pulses in different directions, the apparatus can be equipped with three or more electrodes. For example, three electrodes A, B, and C can be placed, and voltage pulses can be applied in three directions between A and B, B and C, and A and C. In addition, two pairs of (four) electrodes can be placed to apply orthogonal voltage pulses.

Furthermore, the apparatus can be equipped with a system for driving the electrodes to apply voltage pulses in different directions. For example, by rotating the electrodes in the reaction solution, voltage pulses can be applied from a number of different directions. When applying voltage pulses from different directions, the direction of application can be at any angle.

The apparatus for carrying out the invention includes (c) a means for counting agglutinates formed by agglutination of carrier particles carried in said space and/or unagglutinated carrier particles, based on their three-dimensional information as an indicator. The counting means can be mounted in the space mentioned above. Alternatively, counting can be carried out after the reaction solution carried in the space is removed from the space and then introduced into the counting means.

Measuring means based on the Coulter principle or laser diffraction scattering method can be used as means for counting agglutinated or unagglutinated carrier particles using their three-dimensional information as an indicator. For the Coulter principle, for example, a reaction solution in the aforementioned space is introduced into an aperture equipped with electrodes for the Coulter principle to perform the necessary analysis. The size of the aperture can be adjusted appropriately based on standards as described above.

A system of switching and using multiple apertures with different sizes can be employed depending on the diameter of particles used for the reagent or the predicted proportion of agglutinated particles. Furthermore, the apparatus can be equipped with an aperture for counting blood cell components separately from carrier particles. For example, the apparatus of the present invention can be equipped with a flow switching system for guiding the reaction solution into multiple apertures. Furthermore, it is possible to combine a system for automatically selecting the flow path based on the types of reagents or the predicted percentage of agglutinated particles. Alternatively, the apparatus for carrying out the present invention can be equipped with a system for automatically adjusting the detection sensitivity by switching aperture sizes. An example of the system for adjusting the detection sensitivity is a system in which an analysis is initially performed using a larger aperture size, and if the proportion of aggregated particles is predicted to be small, the aperture is switched to a smaller one. When using the laser diffraction scattering method, the reaction solution can be similarly introduced into optical cells for analysis and then analyzed.

In the present invention, three-dimensional information can be obtained after carrier particles that have undergone pearl chain formation in an electric field are redispersed as necessary. The apparatus for carrying out the present invention can be equipped with a system for redispersing carrier particles. Carrier particles can be redispersed by dilution or ultrasonication.

The above-mentioned components (a) to (c) constituting the apparatus for carrying out the present invention can be positioned in a single continuous flow path. Alternatively, the assay method of the present invention can also be carried out by configuring each of the components as a discrete space, and transferring the reaction solution among the components.

Additional systems for carrying out the above-mentioned measurement method can be combined with the apparatus for carrying out the present invention. Additional systems that can be combined with the apparatus of the present invention are exemplified below.

system for dividing samples
system for diluting samples
system for recording measurement results
system for displaying measurement results
system for printing measurement results

All prior art references cited herein are incorporated herein by reference.

[Examples]

Herein below, the present invention will be specifically described with reference to

Examples.

[Reference Experiment]

(1) Measurement of the volume of blood cell components included in whole blood
Whole blood samples were diluted with physiological saline and measured on Multisizer III (manufactured by Beckman Coulter).
(2) Results
The results are shown in Fig. 1. The whole blood components had a volume of approximately 32 $\mu m^3$ to 85 $\mu m^3$ and the average particle diameter was 4 $\mu m$ to 5.5 $\mu m$. Table 1 shows the relationship between the size of insoluble carrier particles and the volume of agglutinates formed by these particles. This shows that when whole blood is assayed as a sample, the size of particles that are less affected by whole blood components is a volume of 30 $\mu m^3$ or less. Generally, particles with a diameter of 2.4 $\mu m$ or less can be used. 1.0 $\mu m$ to 2.0 $\mu m$ or less is preferred and 1.4 to 1.8 $\mu m$ is most preferred.

Table 1A

| Particle diameter $\mu m$ | Volume of agglutinate ($\mu m^3$) | | | |
|---|---|---|---|---|
| | Unagglutinated | 4-particle agglutinate | 6-particle agglutinate | 8-particle agglutinate |
| 1.0 | 0.52 | 2.09 | 3.14 | 4.19 |
| 1.2 | 0.90 | 3.62 | 5.43 | 7.23 |

(continued)

| Particle diameter μm | Volume of agglutinate (μm³) | | | |
|---|---|---|---|---|
| | Unagglutinated | 4-particle agglutinate | 6-particle agglutinate | 8-particle agglutinate |
| 1.4 | 1.44 | 5.74 | 8.62 | 11.49 |
| 1.6 | 2.14 | 8.57 | 12.86 | 17.15 |
| 1.8 | 3.05 | 12.21 | 18.31 | 24.42 |
| 2.0 | 4.19 | 16.75 | 25.12 | 33.49 |
| 2.2 | 5.57 | 22.29 | 33.43 | 44.58 |
| 2.4 | 7.23 | 28.94 | 43.41 | 57.88 |
| 2.6 | 9.20 | 36.79 | 55.19 | 73.58 |
| 2.8 | 11.49 | 45.95 | 68.93 | 91.91 |
| 3.0 | 14.13 | 56.52 | 84.78 | 113.04 |
| 3.2 | 17.15 | 68.59 | 102.89 | 137.19 |
| 3.4 | 20.57 | 82.28 | 123.41 | 164.55 |
| 3.6 | 24.42 | 97.67 | 146.50 | 195.33 |
| 3.8 | 28.72 | 114.87 | 172.30 | 229.73 |
| 4.0 | 33.49 | 133.97 | 200.96 | 267.95 |

Table 1B

| Particle diameter μm | Volume of agglutinate (μm³) | | | |
|---|---|---|---|---|
| | Unagglutinated | 4-particle agglutinate | 6-particle agglutinate | 8-particle agglutinate |
| 5.0 | 65.42 | 261.67 | 392.50 | 523.33 |
| 5.2 | 73.58 | 294.34 | 441.51 | 588.68 |
| 5.4 | 82.41 | 329.62 | 494.44 | 659.25 |
| 5.6 | 91.91 | 367.62 | 551.43 | 735.25 |
| 5.8 | 102.11 | 408.43 | 612.65 | 816.87 |
| 6.0 | 113.04 | 452.16 | 678.24 | 904.32 |
| 6.2 | 124.72 | 498.90 | 748.35 | 997.80 |
| 6.4 | 137.19 | 548.75 | 823.13 | 1097.51 |
| 6.6 | 150.46 | 601.82 | 902.74 | 1203.65 |
| 6.8 | 164.55 | 658.21 | 987.32 | 1316.42 |
| 7.0 | 179.50 | 718.01 | 1077.02 | 1436.03 |
| 7.2 | 195.33 | 781.33 | 1172.00 | 1562.66 |
| 7.4 | 212.07 | 848.27 | 1272.40 | 1696.54 |
| 7.6 | 229.73 | 918.92 | 1378.38 | 1837.85 |
| 7.8 | 248.35 | 993.40 | 1490.09 | 1986.79 |
| 8.0 | 267.95 | 1071.79 | 1607.68 | 2143.57 |

[Example 1]

(1) Preparation of anti-CRP antibody-sensitized latex reagent

Glycine buffer (containing 50 mM glycine, 50 mM sodium chloride, and 0.09% sodium azide; hereinafter abbreviated as GBS) containing 0.15 mg/mL of anti-CRP antibody (manufactured by Shibayagi) was used as an antibody solution for the preparation of a latex reagent. For latex particles, 0.9 mL of GBS was added to 0.1 mL of 1.0 $\mu$m latex (manufactured by Sekisui Chemical, suspension with 10% solid content) to prepare a latex suspension.
1 mL of the antibody solution and the latex suspension were mixed and stirred at 37°C for two hours. Then, sensitized latex was centrifuged and the supernatant was removed. The precipitates were suspended in 2 mL of glycine buffer containing 0.5% bovine serum albumin (0.5% BSA-GBS) to prepare an anti-CRP antibody-sensitized latex reagent was prepared.

(2) Measurement apparatus

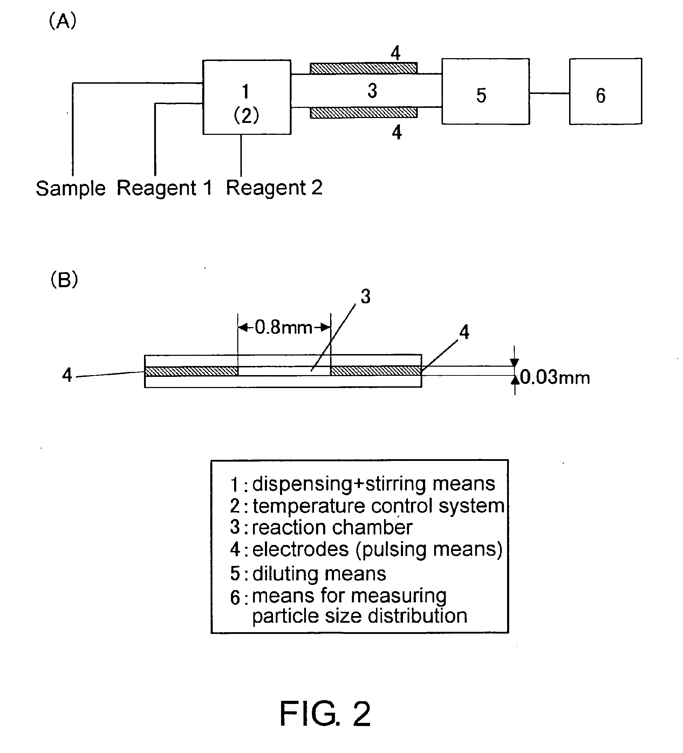

Biologically specific aggregation reactions (antigen-antibody reactions) were measured using the apparatus of Fig. 2(A). The apparatus is equipped with dispensing/stirring chamber temperature controlling system 1, where a sample and Reagent 1 (buffer: applied to a two-reagent system; not needed for a single-reagent system / latex reagent alone) are dispensed and mixed, and Reagent 2 (latex reagent) is further dispensed and mixed. Then, the reaction solution is transferred to reaction chamber (pulsing chamber) 3; and voltage pulses are applied via electrodes 4 for several seconds to several tens of seconds to achieve pearl chain formation. The reaction solution is diluted (dilution chamber 5) after pearl chain formation, and then the state of agglutination of the carriers is measured using particle size distribution analyzer 6 (temperature control systems 1 and 2 were turned off).
Fig. 2(B) is a diagram showing a sectional view of the pulsing chamber. The distance between electrodes is 0.8 mm; electrode thickness is 0.03 mm; and electrode length is 20 mm.

(3) Measurement method

Whole blood samples of healthy subjects were used as samples to carry out the measurement. 2.0 $\mu$L of the sample and 18 $\mu$L of GBS were mixed. 2.0 $\mu$L of this mixture and 2.0 $\mu$L of the anti-CRP antibody-sensitized latex reagent mentioned above were placed in a microtube and mixed. This was immediately injected into a reaction cell equipped with electrodes, and an alternating voltage (rectangular wave) with a frequency of 400 KHz was applied at an electrolytic intensity of 37 Vp-p ($\pm$18.5 V) for 60 seconds to cause pearl chain formation. After this 60-second voltage application, the electric field was immediately removed and the reaction solution was diluted in physiological saline. The size distribution of the latex particles was measured using Multisizer III, and the agglutination rate (AR) of latex was determined by the following formula.

$$AR = \text{(number of particles forming agglutinates of two or more particles)} / \text{(total number of particles)} \times 100\ (\%)$$

(4) Result

The results are shown in Fig. 3.

[Comparative Example 1]

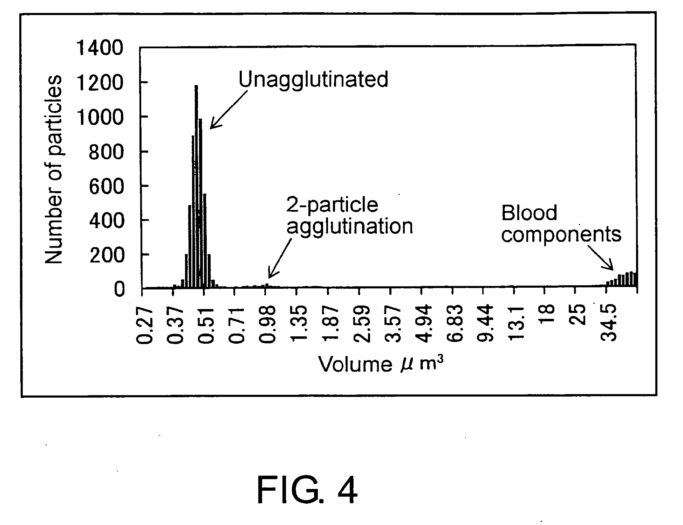

The experiment was performed as in Example 1 using the samples and anti-CRP-sensitized latex reagent of Example 1, except that the alternating voltage (rectangular wave) was set to 0 V. The results are shown in Fig. 4.
Figs. 3 and 4 show that the particle size distribution of latex agglutinates can be measured without influence of whole blood components. In the present invention which accelerates antigen-antibody reactions by pearl chain formation, unagglutinated particles and agglutinates formed by two and three particles were detected as shown in Fig. 3. The agglutination rate was calculated as: AR = 12.6% and CRP value = 0.10 mg/dL. In the comparative example in which pearl chain formation was not performed, AR = 1.5% but CRP was not detectable because there is no difference with the blank value. This shows that compared to conventional methods, the present invention enables rapid and highly sensitive measurements with whole blood samples as well.

[Comparative Example 2]

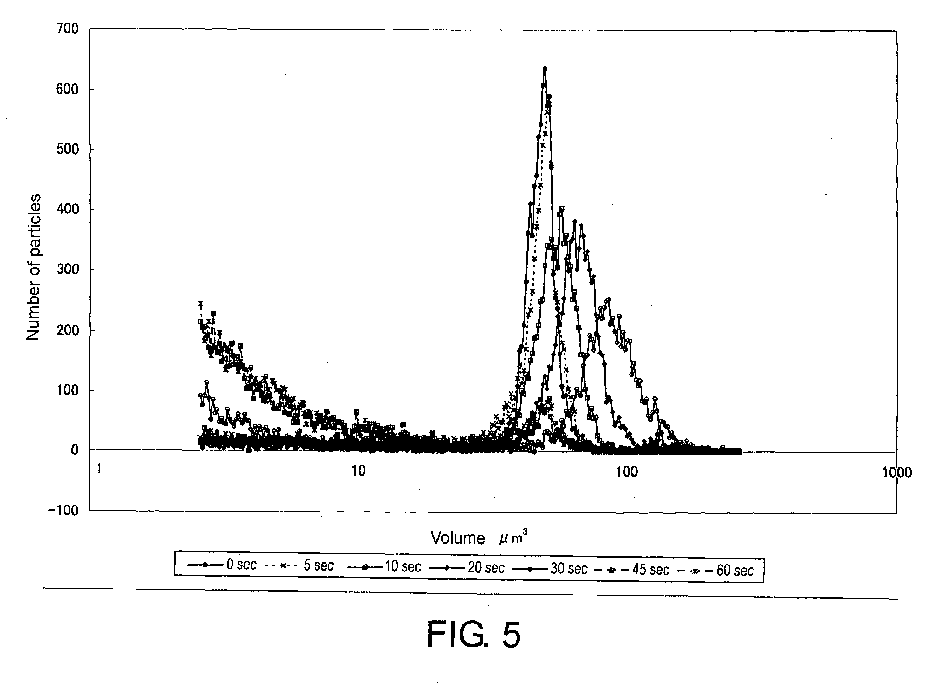

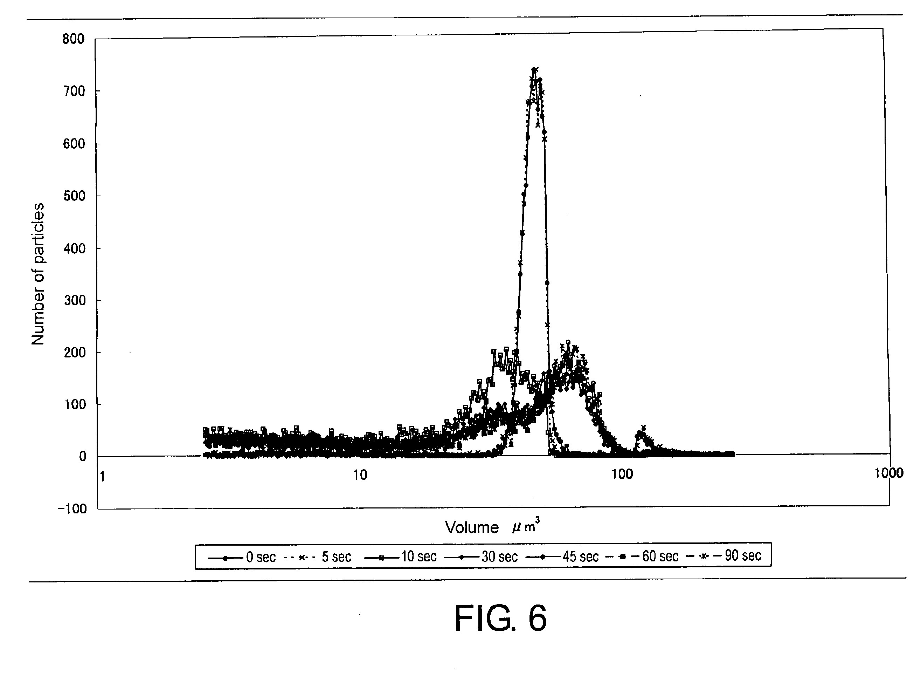

The effect of disruption of blood cell components was compared. 20 μL of whole blood sample and 180 μL of GBS were collected in a microtube and mixed. This mixture was then incubated at 60°C and 80°C for a given period of time. Immediately thereafter, the particle size of the sample was analyzed using a blood cell counter (Multisizer III, manufactured by Beckman Coulter) composed of polystyrene latex beads. The results are shown in Figs. 5 and 6.
As shown in the figures, when the incubation was performed at 80°C for more than 30 seconds, the blood cells started to disrupt. The peak for blood cell-constituting components produced by the disruption was present near approximately 3 $\mu m^3$. This signal was further increased when the incubation time was changed to 45 seconds.

Industrial Applicability

The measurement methods or the measurement apparatus of the present invention are useful for measuring all types of affinity substances that are present in a medium containing blood cell components. Specifically, for example, information useful for diagnosing various diseases can be obtained by analyzing whole blood samples. More specifically, hormones, tumor markers, enzymes, pharmaceutical agents, infectious pathogens, or antibodies against them are routinely measured in medical institutions. These target components are all included in the affinity substances in the present invention.

**Claims**

1. A method for measuring an affinity substance, which comprises the steps of (1) to (3) or (1') to (3'):

    (1) applying a voltage pulse to a reaction solution in which a target affinity substance is mixed with carrier particles which are bound to a binding partner having an activity to bind to the target affinity substance;
    (2) counting either or both of agglutinates of carrier particles formed due to binding of the target affinity substance, and/or unagglutinated carrier particles which are not bound to the target affinity substance, based on their three-dimensional information as an indicator; and
    (3) determining the level of the target substance based on either or both of the level of agglutinate formation and/or the level of unagglutinated carrier particles; or

    (1') applying a voltage pulse to a reaction solution in which an agglutination reagent component is mixed with a target affinity substance and carrier particles which are bound to a binding partner having an activity to bind to the target affinity substance, wherein the carrier particles agglutinate due to the agglutination reagent, and the agglutination is inhibited by the target affinity substance;
    (2') counting either or both of agglutinates of carrier particles formed due to binding of the agglutination reagent, and/or carrier particles whose agglutination is inhibited by binding of the target affinity substance, based on their three-dimensional information as an indicator; and
    (3') determining the level of the target substance based on either or both of the level of agglutinate formation and/or the level of unagglutinated carrier particles,

    wherein the target affinity substance is included in a medium comprising blood cell components,
    wherein step (1) or (1') is carried out in the presence of said medium, and wherein the carrier particles in step (1) or (1') are of a size that yields agglutinates having a volume that can be distinguished from blood cell components.

2. The method of claim 1, wherein the volume that can be distinguished from blood cell components differs from a volume of 40 $\mu m^3$ to 100 $\mu m^3$ by at least 10% or more.

3. The method of claim 2, wherein the average diameter of the carrier particles is 0.5 μm to 2.4 μm or 6 μm to 20 μm.

4. The method of claim 3, wherein the average diameter of the carrier particles is 1 μm to 2 μm.

5. The method of claim 1, wherein the voltage pulse is applied under a condition that does not substantially disrupt the blood cell components included in the sample.

6. The method of claim 5, wherein the voltage pulse is an alternating voltage which gives an electrolytic intensity of 10 V/mm to 50 V/mm.

7. The method of claim 6, wherein the voltage pulse is an alternating voltage which gives an electrolytic intensity of 15 V/mm to 30 V/mm.

8. The method of claim 1, wherein the voltage pulse is an alternating voltage and its frequency is 100 KHz to 10 MHz.

9. The method of claim 8, wherein the voltage pulse is an alternating voltage and its frequency is 400 KHz to 1 MHz.

10. The method of claim 1, wherein the three-dimensional information of the agglutinates or carrier particles is physically measured in step (2) or (2').

11. The method of claim 10, wherein a method of physically measuring the three-dimensional information is selected from the group consisting of electric resistance method, laser diffraction/scattering method, and three-dimensional image analysis method.

12. The method of claim 1, wherein the carrier particles are counted after the electric field is terminated in step (2) or (2').

13. The method of claim 12, which further comprises the step of diluting the carrier particles after the electric field is terminated in step (2) or (2').

14. The method of claim 1, wherein the voltage pulse is applied multiple times.

15. The method of claim 14, which comprises the steps of applying a voltage pulse, dispersing the carrier particles, and then applying a subsequent voltage pulse.

16. The method of claim 14, wherein the multiple voltage pulses are in different directions.

17. The method of claim 1, wherein the concentration of the carrier particles in the reaction solution is 0.1 to 1 w/v%.

18. The method of claim 17, wherein the concentration of the carrier particles in the reaction solution is 0.2 to 0.5 w/v%.

FIG. 1

(A)

Sample  Reagent 1  Reagent 2

(B)

1 : dispensing+stirring means
2 : temperature control system
3 : reaction chamber
4 : electrodes (pulsing means)
5 : diluting means
6 : means for measuring
    particle size distribution

# FIG. 2

**FIG. 3**

FIG. 4

FIG. 5

EP 1 930 725 A1

FIG. 6

EP 1 930 725 A1

| **INTERNATIONAL SEARCH REPORT** | International application No. |
|---|---|
| | PCT/JP2006/319537 |

A.  CLASSIFICATION OF SUBJECT MATTER
*G01N33/543*(2006.01)i, *G01N33/49*(2006.01)i

According to International Patent Classification (IPC) or to both national classification and IPC

B.  FIELDS SEARCHED

Minimum documentation searched (classification system followed by classification symbols)
G01N33/543, G01N33/49

Documentation searched other than minimum documentation to the extent that such documents are included in the fields searched
    Jitsuyo Shinan Koho      1922-1996   Jitsuyo Shinan Toroku Koho   1996-2006
    Kokai Jitsuyo Shinan Koho  1971-2006   Toroku Jitsuyo Shinan Koho   1994-2006

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X | WO 2004/111649 A  (Isao KARUBE),<br>23 December, 2004 (23.12.04),<br>& EP 1637884 A | 1-18 |

☐  Further documents are listed in the continuation of Box C.    ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 October, 2006 (16.10.06) | 24 October, 2006 (24.10.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0783928 A
- WO 2004111649 A
- JP H1048214 A
- JP 2002107365 A
- WO 200196868 A

- JP 2004503779 A
- JP H783928 A
- US PA2656508 A
- US 1953 A

**Non-patent literature cited in the description**

- **CAMBIASO CL.** *J. Immunol. Methods,* 1977, vol. 18 (1-2), 33-44
- **MATSUZAWA et al.** *Kagaku to kogyo,* 1983, vol. 36 (4), 206-208

- **MATSUZAWA et al.** *Chemistry and Chemical Industry,* 1983, vol. 36 (4), 206-208